# EUROPÄISCHE PATENTSCHRIFT

(11) **EP 1 108 358 B1**
(45) Veröffentlichungstag und Bekanntmachung des Hinweises auf die Patenterteilung: **07.04.2004**
(21) Anmeldenummer: 99125293.3
(22) Anmeldetag: 18.12.1999
(51) Int. Cl.: A01M 1/20, A61L 9/03

(54) **Vorrichtung zum Verdampfen von flüchtigen Substanzen, insbesondere von Insektiziden und/oder Duftstoffen**
Device for vaporizing volatile substances, in particular insecticides and/or perfumes
Dispositif de vaporisation de substances volatiles, en particulier insecticides et/ou parfums

(43) Veröffentlichungstag der Anmeldung: 20.06.2001
(73) Patentinhaber: C.T.R., Consultoria, Técnica e Representaçoes Lda, 2715-251 Almargem do Bispo (PT)
(72) Erfinder: Queiroz Vieira, Pedro, 2775-178 Parede (PT)
(74) Vertreter: Liebl, Thomas, Dipl.-Ing.

(56) Entgegenhaltungen:
- EP-A- 0 943 344
- EP-A- 0 962 132
- WO-A-98/19526
- WO-A-98/58692

## Beschreibung

Die Erfindung betrifft eine Vorrichtung zum Verdampfen von flüchtigen Substanzen, insbesondere von Insektiziden und/oder Duftstoffen, gemäß dem Oberbegriff des Anspruchs 1.

Derartige Vorrichtungen zum Verdampfen sind allgemein bekannt. So sind beispielsweise Verdampfungsvorrichtungen bekannt, bei denen ein in eine Verdampfungsvorrichtung eingesetztes und mit einem Wirkstoff imprägniertes Plättchen erhitzt wird, um den Wirkstoff zu verdampfen. Weiter ist es auch bekannt, in ein Gehäuse einer Verdampfungsvorrichtung einen eine flüchtige Substanz enthaltenden Behälter einzusetzen. Dieser Behälter umfasst einen Docht, der die zu verdampfende Substanz mittels der Kapillarwirkung aus dem Behälter fördert, wobei das aus dem Behälter ragende Dochtende benachbart zu einem Heizelement, wie z. B einem Keramikblock, angeordnet ist, so dass die Substanz durch die vom Keramikblock abgestrahlte Wärme verdampft und über Lüftungsschlitze im Gehäuse aus diesem in die Umgebung entweichen kann.

Nachteilig bei diesen Verdampfungsvorrichtungen ist, dass hier keine spezielle Anpassung des Verdampfungsgrades an die jeweils vorherrschenden Raumbedingungen bzw. an die jeweiligen unterschiedlichen Sensitivitäten von sich im Raum befindlichen Personen möglich ist. So ist es zum Beispiel bei kleinen Räumen mit ungenügender Luftzufuhr grundsätzlich wünschenswert, dass der Verdampfungsgrad im Gegensatz zu großen und ggf. gut belüfteten Räumen niedriger einstellbar ist, was mit derartigen Verdampfungsvorrichtungen nicht möglich ist. Desweiteren ist insbesondere hinsichtlich der Verdampfung von Insektiziden eine Einstellbarkeit wünschenswert, damit der Verdampfungsgrad individuell entsprechend der Sensitivität von sich im Raum befindlichen Personen eingestellt werden kann. Auch dies ist mit derartigen Vorrichtungen nicht möglich.

Um den Verdampfungsgrad an die jeweiligen Erfordernisse anpassen zu können, ist es bereits bekannt, den Verdampfungsgrad durch Einstellung der Heizleistung zu steuern. Derartige Verdampfungsvorrichtungen sind insgesamt relativ aufwendig herzustellen und damit teuer. Außerdem sind derartige Vorrichtungen störanfällig.

Alternativ dazu ist es bekannt, bei konstant gehaltener Heizleistung zur Einstellung des Verdampfungsgrades den Relativabstand zwischen Heizelement und Docht zu verändern (EP 0 943 344 A1). Die Verdampfungsvorrichtung besteht hier aus einem einen Anschlussstecker aufweisenden Steckerteil, das als Heizelement einen Widerstand aufweist. Das Steckerteil weist ein Gewinde auf und wird in ein Gehäuseteil eingesetzt, in das wiederum ein Behälter für zu verdampfende Substanzen eingesetzt wird. Am Gehäuseteil sind Stiftausnehmungen vorgesehen, in die Raststifte so eingesetzt werden, dass diese in das Gewinde des Steckerteils eingreifen. Dadurch kann der Abstand des mit dem Steckerteil verbundenen Widerstand-Heizelementes relativ zu einem aus dem Behälter ragenden Dochtende durch Verdrehen des Steckerteils verändert werden. In einer Ausgestaltung hierzu kann das Steckerteil zudem exzentrisch im Gehäuseteil gelagert sein, so dass auch hierüber der Relativabstand zwischen dem Dochtende und dem Widerstand-Heizelement je nach gewünschtem Verdampfungsgrad verändert werden kann. Die Einstellung des Verdampfungsgrades ist hier aufgrund des aufwendigen Aufbaus relativ kompliziert. Aufgrund des komplizierten Aufbaus ist ferner die Herstellung einer derartigen Verdampfungsvorrichtung insgesamt relativ teuer.

Aus der DE 297 14 848 U1 ist ein ähnlicher Aufbau bekannt, der sämtliche Merkmale des Anspruchs 1 aufweist. Konkret ist hier das Heizelement durch ein ringförmiges Widerstandselement gebildet, das ein Außengewinde aufweist, mit dem es in einer kreiszylindrischen Führungseinrichtung geführt ist. Die Führungseinrichtung hat ihrerseits ein Innengewinde, das zum Eingriff in das Außengewinde des Widerstandselementes dient. Ferner weist die Führungseinrichtung einen Verstellring auf, der im Gehäuse in einem Schlitz drehbar gelagert ist und mittels dem die Führungseinrichtung manuell gedreht werden kann, wodurch dann auch aufgrund der Gewindeverbindung zwischen der Führungseinrichtung und dem Widerstandselement letzteres nach aufwärts bzw. abwärts verschiebbar ist. Auch ein derartiger Aufbau ist bauteiltechnisch sehr aufwendig und daher kompliziert, was die Herstellung insgesamt teuer macht. Damit ist auch die Einstellung des Verdampfungsgrades relativ aufwendig.

Eine andere Art von Verdampfungsvorrichtung, bei der die Heizleistung konstant bleibt und bei der der Relativabstand des Dochtes zum Heizelement zur Anpassung des Verdampfungsgrades einstellbar ist, ist aus der WO 98/19526 bekannt. Die Verdampfungsvorrichtung umfasst hier ein Gehäuse, in das ein einen Docht aufweisender Behälter einschraubbar ist. Der Behälter ist hier über eine Buchse mit einem Schwenkarm verbunden, der in einer radial verlaufenden und gegen die Horizontale geneigten Schlitzführung in der Gehäusewand geführt ist. Durch die Kopplung des Schwenkarms mit dem Behälter wird bei einem radialen Verdrehen des Schwenkarms der Behälter relativ zum Gehäuse in Axialrichtung angehoben, wodurch das aus dem Behälter ragende Dochtende relativ zum ortsfesten Heizelement verlagerbar ist. Insgesamt handelt es sich hier um einen relativ aufwendigen und komplizierten Aufbau mit einer Vielzahl von zusätzlichen Bauteilen, so dass eine derartige Verdampfungsvorrichtung auch teuer in der Herstellung ist.

Es ist daher Aufgabe der Erfindung, eine alternative Vorrichtung zum Verdampfen von flüchtigen Substanzen, insbesondere von Insektiziden und/oder Duftstoffen, zu schaffen, die einfach aufgebaut und preiswert herstellbar ist und bei der der Verdampfungsgrad auf einfache Weise an die jeweiligen Erfordernisse anpassbar ist.

Diese Aufgabe wird gelöst mit den Merkmalen des Anspruchs 1.

Gemäß Anspruch 1 ist die Heizeinrichtung als am Gehäuse relativ dazu in Dochtachsenlängsrichtung längsverschiebbar gelagerte Linearführungseinrichtung ausgebildet.

Vorteilhaft ist die Heizeinrichtung hier in einer Doppelfunktion gleichzeitig auch als Linearführungseinrichtung ausgebildet, die am Gehäuse so gelagert ist, dass der Verdampfungsgrad durch deren Längsverschiebung in Dochtachsenlängsrichtung auf einfache Weise einstellbar ist.

Desweiteren wird durch diese Funktionsintegration auch ein insgesamt platzsparender und kompakter Aufbau ohne nennenswerten Bauteil- und Materialaufwand erreicht, da die ohnehin vorhandenen Bauteile auch dazu genutzt werden, um eine individuelle Anpassung des Verdampfungsgrades vornehmen zu können. Eine derartige Verdampfungsvorrichtung ist somit insbesondere auch für beengte Einbausituationen geeignet.

Der Verdampfungsgrad kann somit insgesamt auf einfache Weise an die jeweiligen Erfordernisse individuell angepasst werden, wobei zudem die Bedienung bei gleichzeitig hoher Funktionssicherheit einfach vorzunehmen ist.

Weitere Vorteile des erfindungsgemäßen Aufbaus werden in Verbindung mit den Unteransprüchen ersichtlich:

Grundsätzlich gibt es verschiedene Möglichkeiten die Heizeinrichtung mittels einer Linearführung als Linearführungseinrichtung in Dochtachsenlängsrichtung längsverschiebbar am Gehäuse zu lagern. In einer nach Anspruch 2 bevorzugten Ausführungsform ist die als Linearführungseinrichtung ausgebildete Heizeinrichtung mittels einer Klemmverbindung und/oder einer überdrückbaren Rastverbindung am Gehäuse festlegbar. In einer besonders bevorzugten Ausführungsform ist die Linearführungseinrichtung dabei mittels einer überdrückbaren Klemmverbindung in Dochtachsenlängsrichtung verschiebbar, wobei gleichzeitig eine überdrückbare Rastverbindung vorgesehen ist, mittels der zum Beispiel eine sichere Festlegung in bestimmten Einstellbereichen möglich ist. Damit wird eine größtmögliche Funktionssicherheit dahingehend erreicht, dass der Verdampfungsgrad zum einen durch einfaches Überdrücken der Festlegeverbindung ggf. stufenlos einstellbar ist, während auf der anderen Seite auch sichergestellt ist, dass die Linearführungseinrichtung in der einmal eingestellten Position sicher gehalten wird.

In einer bevorzugten konkreten Ausführungsform weist die Linearführungseinrichtung nach Anspruch 3 wenigstens einen in etwa senkrecht zur Dochtachse ausgerichteten Führungsarm auf, der mit einem Führungsarmende für einen Zugang von der Gehäuseaußenseite her in einer Schlitzausnehmung der Gehäusewand angeordnet ist. Ein derartiger Führungsarm ist an der als Heizeinrichtung ausgebildeten Linearführungseinrichtung ohne nennenswerten Aufwand preiswert ausbildbar. Desweiteren kann ein derartiger Führungsarm durch seine Positionierung in der Schlitzausnehmung als Anzeige auf den gerade eingestellten Abstand der Heizeinrichtung zum Dochtende dienen, ggf. in Verbindung mit einer Skala am die Schlitzausnehmung umgebenden Gehäuserandbereich. Auf der anderen Seite ist über eine derartige Schlitzausnehmung auch der Führungsarm für eine einfache Bedienung gut zugänglich.

Weiter vorteilhaft ist der wenigstens eine Führungsarm dabei in der Schlitzausnehmung zwischen zwei Endanschlagpositionen der Gehäusewand längsverschiebbar gehalten. Für eine vorteilhafte gezielte und kontrollierte Führung der Linearführungseinrichtung ist in einer bevorzugten Ausführungsform nach Anspruch 4 vorgesehen, dass die Linearführungseinrichtung zwei an gegenüberliegenden Gehäuseseiten gelagerte Führungsarme aufweist. Damit wird die Funktionssicherheit der Linearführungseinrichtung insgesamt noch weiter erhöht.

Die Linearführungseinrichtung weist nach Anspruch 5 an jedem Führungsarm ein in etwa parallel zur Dochtachsenlängsrichtung ausgerichtetes erstes Linearführungselement und am Gehäuse ein jedem ersten Linearführungselement entsprechend zugeordnetes zweites Linearführungselement auf. Grundsätzlich kann dabei das erste oder das zweite Linearführungselement als Linearführungsnut und entsprechend umgekehrt das zweite oder erste Linearführungselement als in der Linearführungsnut geführter Linearführungssteg ausgebildet sein. Ein derartiger Aufbau ist auf platzsparende Weise einfach auszubilden und führt somit zu einem insgesamt preiswerten und kompakten Aufbau.

In einer konkreten Ausführungsform gemäß Anspruch 6 ist das erste Linearführungselement als Linearführungsnut und das zweite Linearführungselement als in der Linearführungsnut geführter Linearführungssteg ausgebildet. Für eine besonders einfache und funktionssichere Festlegung bei gleichzeitiger einfacher Verschiebemöglichkeit der Linearführungseinrichtung in unterschiedlichen Einstellpositionen ist dabei der Linearführungssteg mit einem Klemmschluss in der Linearführungsnut geführt. Gemäß einer weiteren vorteilhaften Ausgestaltung der Erfindung sind am Linearführungssteg entsprechend unterschiedlicher Längsrichtungsabstandseinstellungen beabstandete Rastausnehmungen ausgebildet, in die ein in der Linearführungsnut angeordneter Rastvorsprung zur überdrückbaren Festlegung der Linearführungseinrichtung in einem bestimmten Abstand zum Dochtende eingreift. Damit kann die Funktionssicherheit insgesamt nochmals weiter erhöht werden.

Bevorzugt ist gemäß Anspruch 7 der wenigstens eine in etwa senkrecht zur Dochtachsenlängsrichtung ausgerichtete Führungsarm der als Linearführungseinrichtung ausgebildeten Heizeinrichtung mit einem elektrischen Heizelement gekoppelt. Ein derartiges Heizelement kann zum Beispiel durch einen elektrischen Widerstand gebildet sein oder als in einer für Verdampfungsvorrichtungen bevorzugten Ausführungsform als Keramikblock ausgebildet sein. Mit einem derartigen elektrischen Heizelement ergibt sich insgesamt ein guter Wirkungsgrad der gesamten Verdampfungsvorrichtung.

In einer konkreten, bevorzugten Ausführungsform gemäß Anspruch 8 ist das elektrische Heizelement als ein mit elektrischen Leitungen gekoppelter Keramikblock ausgebildet. Der Keramikblock liegt über wenigstens einen Steg auf einer mit dem wenigstens einen Führungsarm verbundenen Trägerplatte auf und ist dort vorzugsweise mittels wenigstens einem elastisch angebundenen und in Richtung Keramikblock vorgespannten Rastarm, der den Keramikblock seitlich L-förmig umgreift, haltbar. Durch die Auflagestege, die vorzugsweise aus Kunststoff ausgebildet sind, wird erreicht, dass insgesamt lediglich kleinflächige Wärmebrücken zu der Trägerplatte ausgebildet werden. Damit wird die Wärmeabstrahlung des Keramikblocks in Richtung auf den Docht hin optimiert. Durch die Rastarme wird dabei eine sichere Halterung des Keramikblocks auf der Trägerplatte erreicht, indem dieser dort einfach einclipsbar ist. Vorzugsweise sind für eine besonders sichere Halterung hier vier Rastarme auf der Trägerplatte angeordnet. In einer besonders bevorzugten Ausführungsform mit zwei gegenüberliegenden Führungsarmen ist die Trägerplatte dabei in etwa in einem mittleren Bereich der beiden Führungsarme vorzugsweise in etwa in der Gehäusemitte angeordnet.

Ferner ist im Keramikblock und entsprechend in der Trägerplatte eine Dochtaussparung ausgebildet, durch die hindurch das Dochtende entsprechend der jeweils gewählten Abstandsposition zum Dochtende führbar ist. Die Dochtaussparung kann dabei zum Beispiel als durchgehendes Durchgangsloch, beispielsweise kreisrund, oder durch eine randseitig senkrecht durchgehende Einbuchtung gebildet sein.

In einer weiteren vorteilhaften Ausgestaltung der Erfindung ist gemäß Anspruch 9 vorgesehen, dass an der Linearführungseinrichtung eine von außen durch eine entsprechende Gehäuseausnehmung sichtbare Abstands- und/oder Betriebsanzeige angeordnet ist. Als Abstands- und/oder Betriebsanzeige ist vorzugsweise eine elektrische Leuchte vorgesehen, zum Beispiel eine Glimmlampe. Damit wird auf einfache Weise erreicht, dass von der Außenseite stets ersichtlich ist, ob sich die Verdampfungsvorrichtung in Betrieb befindet, was insbesondere in Verbindung mit einem gegebenenfalls vorhandenen Ein- und Ausschalter vorteilhaft ist. Gleichzeitig kann damit auch die Position der Linearführungseinrichtung, zum Beispiel in Verbindung mit einer im Bereich der Gehäuseausnehmung angeordneten Skala auf einfache Weise abgelesen werden.

Grundsätzlich kann die Linearführungseinrichtung in unterschiedlichen Einstellund damit Abstandspositionen zum Dochtende stufenlos oder in Intervallen festgelegt werden. In einer bevorzugten Ausführungsform gemäß Anspruch 10 ist die Linearführungseinrichtung zwischen zwei durch gehäuseseitige Anschläge gebildeten Endanschlagpositionen verfahrbar. Dabei ist die Linearführungseinrichtung in einer ersten Endanschlagposition für einen maximalen Verdampfungsgrad unmittelbar im Bereich des Dochtendes angeordnet, während die Linearführungseinrichtung in einer zweiten Endanschlagposition für einen minimalen Verdampfungsgrad beabstandet oberhalb eines in Dochtachsenlängsrichtung gesehen oberen Dochtendes angeordnet ist. Die Linearführungseinrichtung ist dabei vorzugsweise auch in Zwischenpositionen zwischen den beiden Endanschlagpositionen festlegbar. Damit ergibt sich eine gute und individuelle Anpassbarkeit des Verdampfungsgrades an die jeweiligen Erfordernisse.

Auch das Gehäuse selbst kann jeweils entsprechend den unterschiedlichen Ausführungsformen auf unterschiedliche Art und Weise ausgeführt sein. In einer gemäß Anspruch 11 vorteilhaften Ausführungsform weist das Gehäuse Lüftungsschlitze auf und ist mehrteilig aus einer vorderen Gehäuseschale und einer hinteren Gehäuseschale aufgebaut, die miteinander vorzugsweise über entsprechend zugeordnete Rastelemente einer Rastverbindung lösbar verbindbar sind. Die hintere Gehäuseschale ist wiederum durch zwei Gehäusehalbschalen gebildet, die miteinander vorzugsweise ebenfalls über entsprechend zugeordnete Rastelemente einer Rastverbindung lösbar verbindbar sind. Die Trennebene der Gehäusehalbschalen der hinteren Gehäuseschale liegt dabei in einer bevorzugten Ausführungsform in etwa in einem mittleren Längsbereich der hinteren Gehäuseschale. Vorzugsweise ist die Linearführungseinrichtung dabei nach der Montage bzw. Verbindung der Gehäusehalbschalen in Dochtachsenlängsrichtung längsverschiebbar am Gehäuse gelagert. Durch eine derartige Teilung der hinteren Gehäuseschalen in zwei Gehäusehalbschalen wird somit erreicht, dass die Linearführungseinrichtung auf einfache Weise montiert werden kann. So ist es zum Beispiel möglich, die Führungsnuten bei zwei gegenüberliegenden Führungsarmen zuerst an einem Führungssteg einer ersten Gehäusehalbschale einzufädeln und anschließend auf diese vormontierte Baugruppe die zweite Gehäusehalbschale aufzustecken, wobei dann der Führungssteg dieser zweiten Gehäusehalbschale entsprechend in die noch freie Führungsnut des freien Führungsarmes eingreift. Damit ergibt sich eine wesentliche Montagevereinfachung. Die Lüftungsschlitze sind hier vorteilhaft an einem dem Dochtende benachbarten Gehäusebereich ausgebildet, so dass die verdampften Substanzen unmittelbar aus dem Gehäuse heraus in die Umgebung entweichen können.

Grundsätzlich ist es bei dem erfindungsgemäßen Aufbau unproblematisch, dass die Heizeinrichtung, die in einer Doppelfunktion als Linearführungseinrichtung ausgebildet ist, mit elektrischen Leitungen gekoppelt ist, da die Verschiebungswege der Linearführungseinrichtung insgesamt nicht so groß sind, dass hier ein übermäßiger Aufwand zum Ausgleich dieser Verschiebungswege über entsprechende Leitungslängen erforderlich wäre. Um jedoch unter anderem die Leitungen von dem Behälter aufnehmenden Gehäuseinnenraum abzuschirmen, ist in einer nach Anspruch 12 besonders bevorzugten und vorteilhaften Ausführungsform vorgesehen, dass die hintere Gehäuseschale im montierten Zustand zur Ausbildung eines Kabelkanals in einem Bereich unterhalb der Linearführungseinrichtung doppelwandig ausgebildet ist. In einem derartigen Kabelkanal können die Leitungen von der Heizeinrichtung ausgehend bis zu einem Anschlussbereich am Gehäuse, zum Beispiel einem Anschlussstecker verstaut werden, so dass diese vom Gehäuseinnenraum abgeschirmt sind. Dadurch wird unter anderem auch erreicht, dass die elektrischen Leitungen beim Einsetzen eines Behälters in den Gehäuseinnenraum nicht stören.

In einer hierzu vorteilhaften Ausgestaltung umfasst gemäß Anspruch 13 ein im doppelwandigen Gehäusebereich die Gehäuseaußenwand bildende Rückwand der hinteren Gehäuseschale eine Ausnehmung zur Aufnahme eines Anschlusssteckers zum Anschluss an eine Steckdose. In diese Ausnehmung ist der Anschlussstecker, vorzugsweise bei der Verbindung der Gehäusehalbschalen, vorzugsweise über eine Nut-Steg-Paarung, einsetzbar. Die elektrischen Leitungen sind hier im Kabelkanal vom Anschlussstecker ausgehend zu der als Linearführungseinrichtung ausgebildeten Heizeinrichtung geführt. In einer besonders vorteilhaften Ausführungsform können dabei die Leitungen im Kabelkanal gegebenenfalls so verklemmt werden, dass die Leitungen bei maximalem Abstand der Linearführungseinrichtung vom Dochtende in der zweiten Endanschlagposition in etwa gespannt sind. Damit ergibt sich auch hinsichtlich der Leitungslängen kein Problem durch die Längsverschiebbarkeit der als Linearführungseinrichtung ausgebildeten Heizeinrichtung.

Alternativ dazu ist es auch möglich, dass in der Rückwand anstelle einer Ausnehmung für einen Anschlussstecker auch lediglich eine Kabeldurchführung vorgesehen ist, so dass das Gehäuse auch mit einem enfernt vom Aufstellungsort in eine Steckdose einsteckbaren Kabel verbunden sein kann.

Für eine besonders einfache und gut zugängliche Einsetzmöglichkeit des Behälters in das Gehäuse ist gemäß Anspruch 14 vorgesehen, dass in einem unteren Gehäusebereich ein Aufnahmeschlitz oder eine Aufnahmeöffnung vorgesehen ist, über die der Behälter wenigstens teilweise in den Gehäuseinnenraum einführbar und/oder einschraubbar und dort lösbar festlegbar, vorzugsweise einklipsbar, ist.

Die Erfindung wird nachfolgend anhand einer Zeichnung näher erläutert.

Es zeigen:
- Fig. 1: eine schematische Draufsicht auf eine vordere Gehäuseschale,
- Fig. 2: eine schematische Draufsicht auf einen Anschlussstecker, der über elektrische Leitungen mit einer als Linearführungseinrichtung ausgebildeten Heizeinrichtung gekoppelt ist,
- Fig. 3: eine schematische Darstellung zweier eine hintere Gehäuseschale bildenden Gehäusehalbschalen,
- Fig. 4: eine schematische Darstellung der beiden Gehäusehalbschalen gem. Fig. 3 im zusammengefügten Zustand,
- Fig. 5: eine schematische Darstellung einer hinteren Gehäuseschale mit einer daran längsverschiebbar geführten Heizeinrichtung als Linearführungseinrichtung in einer ersten Endanschlagposition,
- Fig. 6: eine schematische Darstellung einer hinteren Gehäusehälfte mit einer daran längsverschiebbar geführten Heizeinrichtung als Linearführungseinrichtung in einer zweiten Endanschlagposition,
- Fig. 7: einen schematischen Querschnitt durch eine fertig montierte Verdampfungsvorrichtung mit einer Draufsicht auf die als Linearführungseinrichtung ausgebildete Heizeinrichtung mit einem Keramikblock,
- Fig. 8: die Darstellung gem. Fig. 8 ohne Keramikblock, und
- Fig. 9: einen schematischen Längsquerschnitt durch eine fertig montierte Verdampfungsvorrichtung mit schematischen strichliert eingezeichnetem Behälter für eine zu verdampfende Substanz.

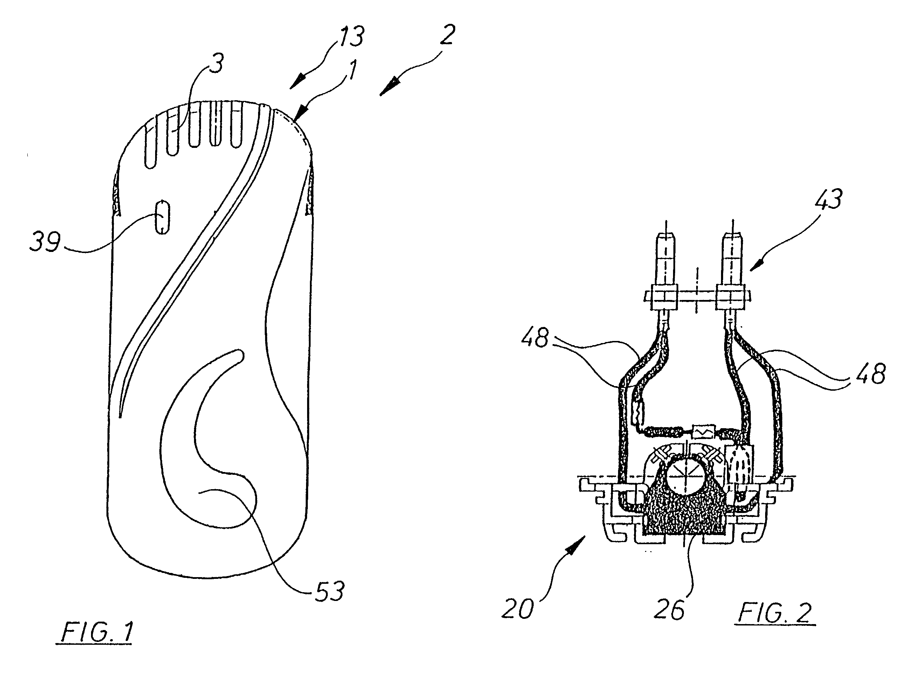

In der Fig. 1 ist schematisch eine Draufsicht auf eine vordere Gehäuseschale 1 einer Verdampfungsvorrichtung 2 schematisch dargestellt. Diese vordere Gehäuseschale 1 weist an einem oberen Ende mehrere Lüftungsschlitze 3 auf, durch die eine verdampfte Substanz in die Umgebung entweichen kann. Ferner ist der vorderen Gehäuseschale 1 auch noch eine hier beispielhaft tropfenförmig ausgebildete Ausnehmung als Füllstandsanzeige 53, zum Beispiel in Verbindung mit durchsichtigen Behälter, ausgebildet.

In der Fig. 2 ist schematisch eine Draufsicht auf einen elektrischen Anschlussstecker 43 gezeigt, der über elektrischen Leitungen 48 mit einer als Linearführungseinrichtung 20 ausgebildeten Heizeinrichtung gekoppelt ist, was nachfolgend in Verbindung mit den weiteren Fig. noch näher erläutert wird.

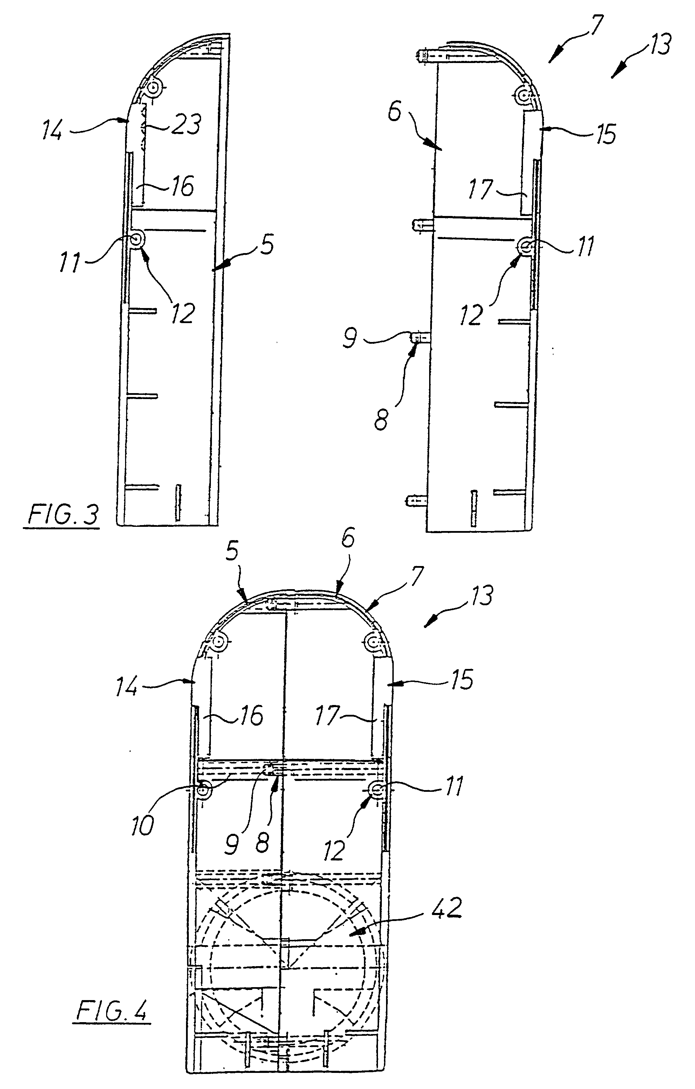

In der Fig. 3 ist eine aus zwei Gehäusehalbschalen 5, 6 bestehende hintere Gehäuseschale 7 dargestellt, die mittels mehrerer Rastverbindungen 8 lösbar miteinander verbindbar sind, wie dies auch aus der Fig. 4 hervorgeht, die die beiden Gehäusehalbschalen 5, 6 im verbundenen Zustand zeigt. Die Rastverbindung 8 ist hier jeweils durch einen Raststift 9 an der Gehäusehalbschale 6 gebildet, der in eine entsprechend zugeordnete Raststiftausnehmung 10 an der Gehäuseschale 5 eingreift, gebildet. Anstelle der hier gezeigten Ausbildung der Raststifte 9 an der Gehäusehalbschale 6, können die Raststifte 9 auch an der Gehäusehalbschale 5 ausgebildet sein. Ebenso ist es möglich, dass Raststifte 9 und Raststiftausnehmungen 10 wechselweise sowohl an der Gehäusehalbschale 5 als auch an der Gehäusehalbschale 6 auszubilden. Die Raststifte 9 weisen gegenüber der Raststiftausnehmung 10 vorzugsweise jeweils ein geringes Übermaß auf, so dass diese im in der Fig. 4 dargestellten miteinander verbundenen Zustand eine Klemmverbindung ausbilden.

Wie dies aus den Fig. 3 und 4 weiter ersichtlich ist, sind an den Gehäusehalbschalen 5, 6 der hinteren Gehäuseschale 7 weitere Raststiftausnehmungen 11 ausgebildet, in die entsprechend an der vorderen Gehäuseschale 1 ausgebildete und in der Fig. 1 nicht dargestellte Raststifte einer Rastverbindung 12 auf die eben in Verbindung mit den Rastverbindungen 8 beschriebene Art und Weise einsteckbar sind. Im montierten Zustand bilden die hintere Gehäuseschale 7 und die vordere Gehäuseschale 1 dann ein mehrteiliges Gehäuse 13 der Verdampfungsvorrichtung 2 aus.

Wie dies aus den Fig. 3 und 4 weiter ersichtlich ist, ist in einem in der Bildebene oberen Bereich der Gehäusehalbschalen 5, 6 und damit der hinteren Gehäuseschale 7 jeweils aufeinander in gleicher Höhe gegenüberliegenden Seiten jeweils eine Schlitzausnehmung 14, 15 ausgebildet. Dieser Schlitzausnehmung 14, 15 ist jeweils ein am Gehäuse ausgebildeter Linearführungssteg 16, 17 zugeordnet, der sich von einem oberen Schlitzausnehmungsrandbereich ausgehend bis zu einem sich über den Bereich der unteren Schlitzausnehmungsrand hinaus erstreckenden Bereich erstreckt.

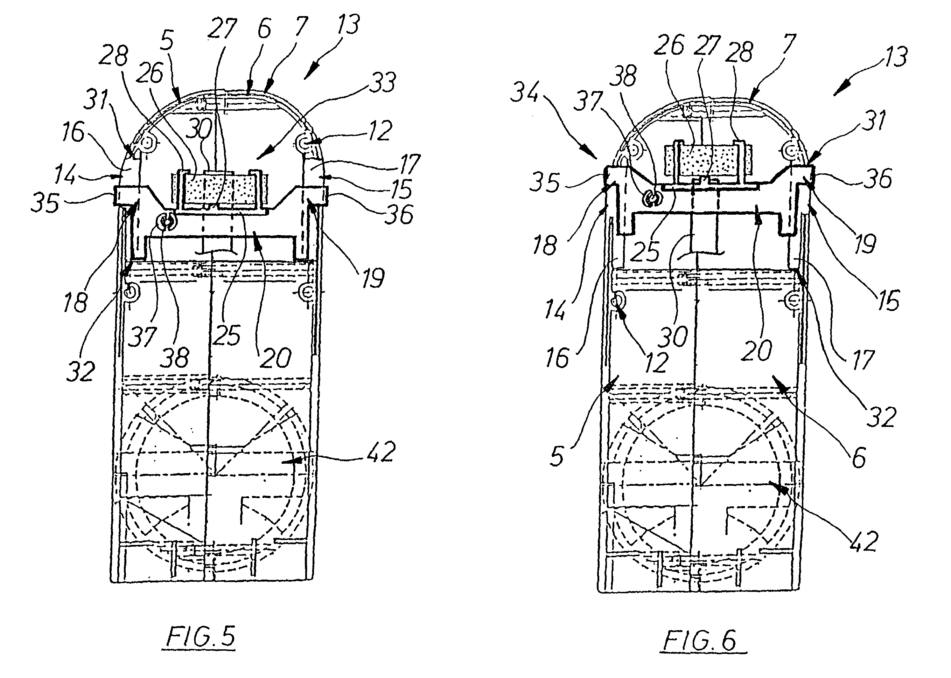

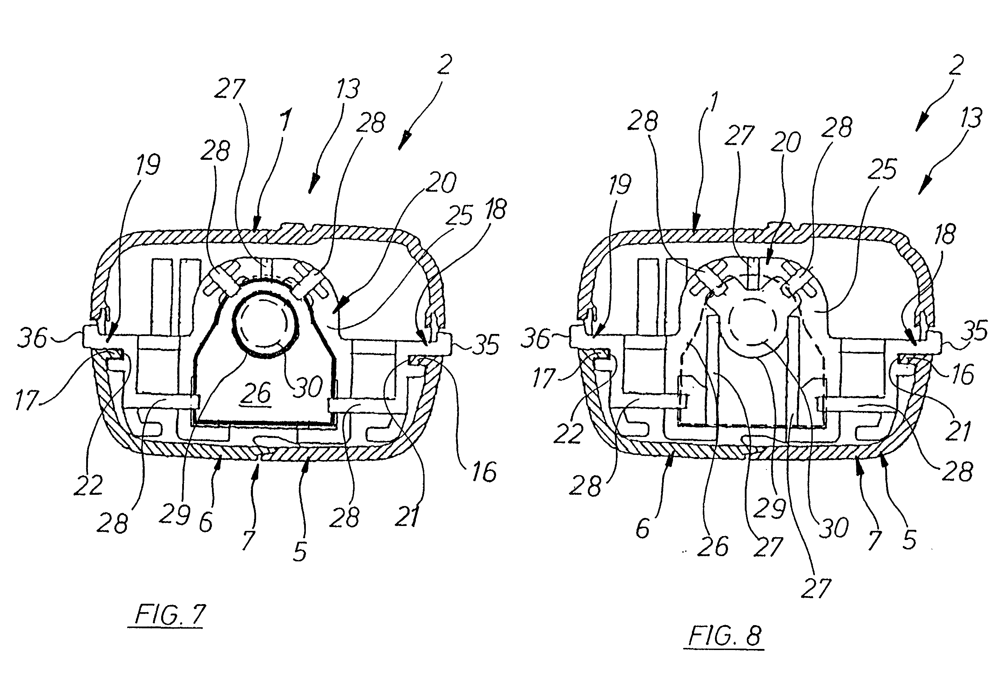

Diesen Linearführungsstegen 16, 17 ist, wie dies insbesondere aus den Fig. 5 und 6 in Verbindung mit den Fig. 7 und 8 ersichtlich ist, jeweils ein Führungsarm 18, 19 einer Linearführungseinrichtung 20 mit einer daran ausgebildeten Linearführungsnut 21, 22 zugeordnet. Die Linearführungsstege 16, 17 sind dabei vorzugsweise mit einem Klemmschluss in den Linearführungsnuten 21, 22 längsverschiebbar geführt.

Wie dies beispielhaft aus der Fig. 3 ersichtlich ist, kann alternativ oder auch zusätzlich zu diesem Klemmschluss an einem oder an beiden Linearführungsstegen 16, 17 jeweils mehrere Rastausnehmungen 23 ausgebildet sein, in die ein entsprechend im Bereich der Linearführungsnuten 21, 22 ausgebildeter Rastvorsprung 24 zur überdrückbaren Festlegung der Linearführungseinrichtung 20 in bestimmten Einstellpositionen eingreifen kann.

Die Linearführungseinrichtung 20 wird hier durch eine Heizeinrichtung gebildet, die, wie dies insbesondere auch aus der Fig. 7 ersichtlich ist, die einen Querschnitt durch eine fertig montierte Verdampfungsvorrichtung 2 zeigt, eine Trägerplatte 25 in einem mittleren Bereich zwischen den beiden Führungsarmen 18, 19 aufweist. Auf dieser Trägerplatte 25 liegt ein Keramikblock 26 als Heizelement auf. In der Fig. 8 ist eine Darstellung entsprechend der Fig. 7 gezeigt, bei der der Keramikblock 26 lediglich strichliert eingezeichnet ist.

Wie dies aus den Fig. 5 bis 8 weiter ersichtlich ist, liegt der Keramikblock 26 über Stege 27 auf der Trägerplatte 25 auf und ist auf dieser Trägerplatte 25 durch vier über den Umfang der Trägerplatte 25 verteilte Rastarme 28 dort gehalten. Die Rastarme 28 sind dabei ebenso wie das Gehäuse 13 und die Führungsarme 18, 19 aus einem Kunststoffmaterial hergestellt und elastisch an der Trägerplatte 25 in Richtung Keramikblock 26 vorgespannt angebunden. Im aufgeklipsten Zustand umgreifen dabei die Rastarme 28 den Keramikblock 26 in etwa L-förmig, wie dies insbesondere aus den Fig. 5 und 6 ersichtlich ist.

Den Fig. 7 und 8 kann weiter entnommen werden, dass der Keramikblock 26 und entsprechend die Trägerplatte 25 ein jeweils senkrecht durchgehendes und miteinander fluchtendes Durchgangsloch 29 aufweisen.

Wie dies in den Fig. 5 und 6 lediglich teilweise und schematisch dargestellt ist, ist bei in das Gehäuse 13 eingesetztem Behälter für eine zu verdampfende Substanz, der einen Docht umfasst, ein aus dem Behälter ragendes Dochtende 30 durch das Durchgangsloch 29 in der Trägerplatte 25 und im Keramikblock 26 hindurchgeführt.

Wie dies aus den Fig. 5 und 6 zudem weiter ersichtlich ist, ist die als Linearführungseinrichtung 20 ausgebildete Heizeinrichtung in Dochtachsenlängsrichtung gesehen zwischen einer in der Fig. 5 dargestellten ersten Endanschlagposition 33 und einer in der Fig. 6 dargestellten zweiten Endanschlagposition 34 verschiebbar. Die erste und zweite Endanschlagposition 33, 34 ist dabei konstruktiv durch gehäuseseitige Anschläge 31, 32 gebildet, die z.B. durch das Zusammenwirken der in Längserstreckungsrichtung gegenüberliegenden Schlitzausnehmungsrandbereiche in Verbindung mit den jeweils zugeordneten Führungsarmenden 35, 36 der Führungsarme 18, 19 gebildet sind. Die beiden Führungsarmenden 35, 36 ragen dabei so durch die Schlitzausnehmungen 14, 15, dass diese von der Gehäuseaußenseite her mit den Fingern leicht greifbar sind.

In der in der Fig. 5 dargestellten ersten Endanschlagposition 33 ist das Dochtende 30 unmittelbar vom Keramikblock 26 umgeben, so dass bei einer derartigen Anordnung ein maximaler Verdampfungsgrad eingestellt ist. Dagegen wird bei der in der Fig. 6 dargestellten zweiten Endanschlagposition, bei der der Keramikblock 26 oberhalb eines Dochtendes 30 angeordnet ist, lediglich ein geringerer Verdampfungsgrad erreicht, da die vom Keramikblock 26 abgegebene Wärme nur teilweise unmittelbar auf das Dochtende 30 einwirkt. Selbstverständlich ist zwischen diesen beiden in den Fig. 5 und 6 dargestellten Extremlagen auch eine Festlegung der Linearführungseinrichtung 20 in Zwischenpositionen gegebenenfalls vorgegeben durch die Rastausnehmungen 23 möglich.

Wie dies aus den Fig. 5 und 6 weiter entnommen werden kann, ist am Führungsarm 18 eine in einem Abschirmgehäuse 37 gehalterte elektrische Leuchte 38 als Abstands- und/oder Betriebsanzeige angeordnet, die einer in der Fig. 1 dargestellten Gehäuseausnehmung 39 zugeordnet ist. Diese Leuchte 38 kann zum einen zum Beispiel in Verbindung mit einem an dem Gehäuse angeordneten Ein- und Ausschalter als Anzeige des Betriebszustandes dienen oder aber auch entsprechend ihrer Position relativ zum Gehäuse in der Gehäuseausnehmung 39 gegebenenfalls in Verbindung mit einer im Randbereich der Gehäuseausnehmung 39 angeordneten Skala als Positionsanzeige bezüglich des Verdampfungsgrades dienen. Alternativ oder zusätzlich dazu kann eine derartige Skalierung auch an der Gehäuseaußenseite im Bereich der Schlitzausnehmungen 14, 15 vorgesehen sein.

Durch den Aufbau der hinteren Gehäuseschale 7 aus den Gehäusehalbschalen 5, 6 ist eine Montage der Linearführungseinrichtung 20 auf einfache Weise möglich. So kann die Linearführungseinrichtung 20 z.B. mit der Linearführungsnut 21 am Linearführungssteg 16 der Gehäusehalbschale 5 eingefädelt werden und anschließend die zweite Gehäusehalbschale 6 auf die Gehäusehalbschale 5 aufgesteckt werden, wobei der Linearführungssteg 17 der Gehäusehalbschale 6 in die Linearführungsnut 22 des Führungsarmes 19 eingreift. Dadurch ist die als Linearführungseinrichtung 20 ausgebildete Heizeinrichtung nach der Verbindung der beiden Gehäusehalbschalen 5, 6 in Dochtachsenlängsrichtung längsverschiebbar am Gehäuse 13 gelagert.

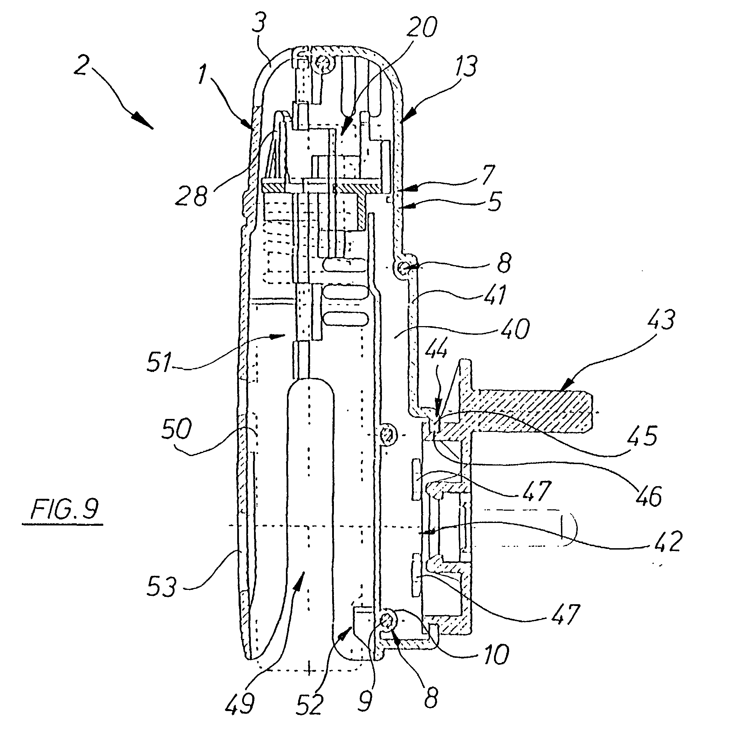

Wie dies insbesondere aus der Fig. 9 ersichtlich ist, die einen schematischen Längsquerschnitt durch eine fertig montierte Verdampfungsvorrichtung 2 zeigt, ist die hintere Gehäuseschale 7 im montierten Zustand in einem Bereich unterhalb der Linearführungseinrichtung 20 unter Ausbildung eines Kabelkanals 40 doppelwandig ausgebildet.

Eine in diesem doppelwandigen Gehäusebereich die Gehäuseaußenwand bildende Rückwand 41 der hinteren Gehäuseschale 7 umfasst hier eine Ausnehmung 42, die strichliert auch in den Fig. 4 bis 6 dargestellt ist. In diese Ausnehmung 42 ist ein Anschlussstecker 43 bei der Verbindung der Gehäusehalbschalen 5, 6 über eine Nut-Steg-Paarung 44 einsetzbar.

Diese Nut-Steg-Paarung 44 umfasst an der Rückwand 41 einen bei verbundenen Gehäusehalbschalen 5, 6 umlaufenden Ringsteg 45, der im montierten Zustand in eine entsprechende Ringausnehmung 46 am Anschlussstecker 43 eingreift. Vorteilhaft können dabei auch noch in die Ausnehmung 42 vorstehende und im Bereich der Rückwand 41 angeformte Klemmarme 47 vorgesehen sein, die eine weitere Verklemmung des Anschlusssteckers 43 in der Rückwand 41 ermöglichen.

In dem Kabelkanal 40 sind hier nicht dargestellte elektrische Leitungen vom Anschlussstecker 43 ausgehend zu dem Keramikblock 26 der als Linearführungseinrichtung 20 ausgebildeten Heizeinrichtung geführt. Ebenfalls kann hier eine elektrische Leitung vom Anschlussstecker 43 ausgehend zu der elektrischen Leuchte 38 geführt sein.

Dabei können die elektrischen Leitungen im Kabelkanal 40 zum Beispiel zwischen den Raststiftausnehmungen 10 der Rastverbindungen 12 und der Innenwand der Rückwand 41 so verklemmt werden, dass die Leitungen bei maximalem Abstand der Linearführungseinrichtung 20 vom Dochtende 30 in der zweiten Endanschlagposition 34 in etwa gespannt sind.

Eine schematische Darstellung des Anschlusssteckers 43 mit den zu der als Linearführungseinrichtung 20 ausgebildeten Heizeinrichtung, insbesondere zum Keramikblock 26 und zur elektrischen Leuchte 38 geführten elektrischen Leitungen 48 ist in der Fig. 2 im unmontierten Zustand schematisch und in einer Draufsicht dargestellt.

Aus der Fig. 9 ist ferner schematisch und strichliert ersichtlich, dass das Gehäuse 13 in einem unteren Gehäusebereich einen Aufnahmeschlitz 49 aufweist, über den ein hier lediglich schematisch und strichliert eingezeichneter Behälter 50 wenigstens teilweise in den Gehäuseinnenraum 51 eingeführt und dort lösbar festgelegt werden kann. Die lösbare Festlegung erfolgt beispielsweise über eine im unteren Aufnahmeschlitzbereich angeordnete Rastverbindung 52.

## Patentansprüche

1. Vorrichtung zum Verdampfen von flüchtigen Substanzen, insbesondere von Insektiziden und/oder Duftstoffen,
mit einem Gehäuse (13), das eine Heizeinrichtung (20) umfasst, und
mit einem mit dem Gehäuse (13) verbindbaren Behälter (50) für eine zu verdampfende Substanz, der einen Docht umfasst, der mit einem aus dem Behälter (50) ragenden Dochtende (30) der Heizeinrichtung (20) zugeordnet ist, wobei die Heizeinrichtung (20) zur Einstellung des Verdampfungsgrades bei mit dem Gehäuse (13) verbundenem Behälter (50) relativ zum Dochtende (30) und damit zum Gehäuse (13) so verlagerbar ist, dass die Heizeinrichtung (20) in Dochtachsenlängsrichtung gesehen in unterschiedlichen Positionen zum Dochtende (30) festlegbar ist,
**dadurch gekennzeichnet,**
**dass** die Heizeinrichtung als am Gehäuse (13) relativ dazu in Dochtachsenlängsrichtung längsverschiebbar gelagerte Linearführungseinrichtung (20) ausgebildet ist.

2. Vorrichtung nach Anspruch 1, **dadurch gekennzeichnet, dass** die als Linearführungseinrichtung (20) ausgebildete Heizeinrichtung mittels einer Klemmverbindung und/oder einer überdrückbaren Rastverbindung (23, 24) am Gehäuse (13) festlegbar ist.

3. Vorrichtung nach Anspruch 1 oder Anspruch 2, **dadurch gekennzeichnet, dass** die Linearführungseinrichtung (20) wenigstens einen senkrecht zur Dochtachse ausgerichteten Führungsarm (18, 19), aufweist, der mit einem Führungsarmende (35, 36) für einen Zugang von der Gehäuseaußenseite her in einer Schlitzausnehmung (14, 15) der Gehäusewand angeordnet ist.

4. Vorrichtung nach Anspruch 3, **dadurch gekennzeichnet, dass** die Linearführungseinrichtung (20) zwei an gegenüberliegenden Gehäuseseiten gelagerte Führungsarme (18, 19) aufweist.

5. Vorrichtung nach Anspruch 3 oder Anspruch 4, **dadurch gekennzeichnet,**
**dass** die Linearführungseinrichtung (20) an jedem Führungsarm (18, 19) ein parallel zur Dochtachsenlängsrichtung ausgerichtetes erstes Linearführungselement (21, 22) aufweist, und
**dass** die Linearführungseinrichtung (20) am Gehäuse (13) ein jedem ersten Linearführungselement (21, 22) entsprechend zugeordnetes zweites Linearführungselement (16, 17) aufweist.

6. Vorrichtung nach Anspruch 5, **dadurch gekennzeichnet,**
**dass** das erste Linearführungselement eine Linearführungsnut (21, 22) und das zweite Linearführungselement ein darin geführter Linearführungssteg (16, 17) ist, und/oder
**dass** am Linearführungssteg (16, 17) entsprechend unterschiedlicher Längsrichtungsabstandseinstellungen beabstandete Rastausnehmungen (23) ausgebildet sind, in die ein im Bereich der Linearführungsnut (21, 22) ausgebildeter Rastvorsprung (24) zur überdrückbaren Festlegung der Linearführungseinrichtung (20) in einem bestimmten Abstand zum Dochtende (30) eingreift.

7. Vorrichtung nach einem der Ansprüche 3 bis 6, **dadurch gekennzeichnet, dass** der wenigstens eine senkrecht zur Dochtachsenlängsrichtung ausgerichtete Führungsarm (18, 19) der als Linearführungseinrichtung (20) ausgebildeten Heizeinrichtung mit einem elektrischen Heizelement (26) gekoppelt ist.

8. Vorrichtung nach Anspruch 7, **dadurch gekennzeichnet,**
**dass** das elektrische Heizelement ein mit elektrischen Leitungen (48) gekoppelter Keramikblock (26) ist, der über wenigstens einen Steg (27) auf einer mit dem wenigstens einen Führungsarm (18, 19) verbundenen Trägerplatte (25) aufliegt und dort mittels wenigstens einem, elastisch angebundenen und in Richtung Keramikblock (26) vorgespannten Rastarm (28), der den Keramikblock (26) seitlich L-förmig umgreift, haltbar ist, und
**dass** im Keramikblock (26) und entsprechend in der Trägerplatte (25) eine Dochtaussparung (29) ausgebildet ist, durch die hindurch das Dochtende (30) aufnehmbar ist.

9. Vorrichtung nach einem der Ansprüche 1 bis 8, **dadurch gekennzeichnet, dass** an der Linearführungseinrichtung (20) eine von außen durch eine entsprechende Gehäuseausnehmung (39) sichtbare Abstandsund/oder Betriebsanzeige angeordnet ist.

10. Vorrichtung nach einem der Ansprüche 1 bis 9, **dadurch gekennzeichnet,**
**dass** die Linearführungseinrichtung (20) zwischen zwei durch gehäuseseitige Anschläge (31, 32) gebildeten Endanschlagpositionen (33, 34) längsverschiebbar ist dergestalt,
**dass** die Linearführungseinrichtung (20) in einer ersten Endanschlagposition (33) für einen maximalen Verdampfungsgrad unmittelbar im Bereich des Dochtendes (30) angeordnet ist, und
**dass** die Linearführungseinrichtung (20) in einer zweiten Endanschlagposition (34) für einen minimalen Verdampfungsgrad beabstandet oberhalb eines in Dochtachsenlängsrichtung gesehen oberen Dochtendes (30) angeordnet ist, wobei die Linearführungseinrichtung (20) auch in Zwischenpositionen zwischen beiden Endanschlagpositionen (33, 34) festlegbar ist.

11. Vorrichtung nach einem der Ansprüche 1 bis 10, **dadurch gekennzeichnet,**
**dass** das Gehäuse (13) Lüftungsschlitze (3) aufweist und mehrteilig aus einer vorderen Gehäuseschale (1) und einer hinteren Gehäuseschale (7) aufgebaut ist, die miteinander über entsprechend zugeordnete Rastelemente (9, 10) einer Rastverbindung (8) lösbar verbindbar sind, und
**dass** die hintere Gehäuseschale (7) durch zwei Gehäusehalbschalen (5, 6) gebildet ist, die miteinander über entsprechend zugeordnete Rastelemente (11) einer Rastverbindung (12) lösbar verbindbar sind dergestalt, dass die Linearführungseinrichtung (20) nach Montage der Gehäusehalbschalen (5, 6) in Dochtachsenlängsrichtung längsverschiebbar am Gehäuse (13) gelagert ist.

12. Vorrichtung nach Anspruch 11, **dadurch gekennzeichnet, dass** die hintere Gehäuseschale (7) im montierten Zustand in einem Bereich unterhalb der Linearführungseinrichtung (20) zur Ausbildung eines Kabelkanals (40) doppelwandig ausgebildet ist.

13. Vorrichtung nach Anspruch 12, **dadurch gekennzeichnet,**
**dass** eine im doppelwandigen Gehäusebereich die Gehäuseaußenwand bildende Rückwand (41) der hinteren Gehäuseschale (7) eine Ausnehmung (42) zur Aufnahme eines Anschlusssteckers (43) zum Anschluss an eine Steckdose umfasst, in der der Anschlussstecker (43) einsetzbar ist dergestalt,
**dass** die elektrischen Leitungen (48) im Kabelkanal (40) vom Anschlussstecker (43) ausgehend zur als Linearführungseinrichtung (20) ausgebildeten Heizeinrichtung geführt sind, wobei die Leitungen (48) im Kabelkanal (40) so verklemmt sind, dass die Leitungen (48) bei maximalem Abstand der Linearführungseinrichtung (20) vom Dochtende (30) in etwa gespannt sind.

14. Vorrichtung nach einem der Ansprüche 11 bis 13, **dadurch gekennzeichnet, dass** das Gehäuse (13) in einem unteren Gehäusebereich einen Aufnahmeschlitz (49) oder eine Aufnahmeöffnung aufweist, über die der Behälter (50) wenigstens teilweise in den Gehäuseinnenraum (51) einführbar und/oder einschraubbar und dort lösbar festlegbar ist.

## Claims

1. Device for vaporizing volatile substances, in particular insecticides and/or perfumes,
with a housing (13) which comprises a heating arrangement (20), and
with a container (50) for a substance to be vaporized, which container (50) can be connected to the housing (13) and comprises a wick which, with a wick end (30) protruding from the container (50), is assigned to the heating arrangement (20), said heating arrangement (20) being displaceable relative to the wick end (30), and thus relative to the housing (13), for adjusting the degree of vaporization when the container (50) is connected to the housing (13), in such a way that the heating arrangement (20), viewed in the longitudinal direction of the wick axis, can be fixed in different positions with respect to the wick end (30),
**characterized in that**
the heating arrangement is designed as a linear guide arrangement (20) mounted on the housing (13) so as to be longitudinally displaceable relative thereto in the longitudinal direction of the wick axis.

2. Device according to Claim 1, **characterized in that** the heating arrangement designed as linear guide arrangement (20) can be fixed on the housing (13) by means of a clamp connection and/or a snap-in catch connection (23, 24).

3. Device according to Claim 1 or Claim 2, **characterized in that** the linear guide arrangement (20) has at least one guide arm (18, 19) which is oriented perpendicular to the wick axis and which, with a guide arm end (35, 36) for access from outside the housing, is disposed in a slotted recess (14, 15) of the housing wall.

4. Device according to Claim 3, **characterized in that** the linear guide arrangement (20) has two guide arms (18, 19) mounted on opposite sides of the housing.

5. Device according to Claim 3 or Claim 4, **characterized in that**
the linear guide arrangement (20) has, on each guide arm (18, 19), a first linear guide element (21, 22) oriented parallel to the longitudinal direction of the wick axis, and
the linear guide arrangement (20) on the housing (13) has a second linear guide element (16, 17) assigned correspondingly to each first linear guide element (21, 22).

6. Device according to Claim 5, **characterized in that**
the first linear guide element is a linear guide groove (21, 22), and the second linear guide element is a linear guide ridge (16, 17) guided in the latter, and/or
catch recesses (23) spaced apart for different settings in the longitudinal direction are provided on the linear guide ridge (16, 17), into which recesses (23) a locking projection (24) formed in the area of the linear guide groove (21, 22) engages with a snap-fit to secure the linear guide arrangement (20) at a defined distance from the wick end (30).

7. Device according to one of Claims 3 to 6, **characterized in that** the at least one guide arm (18, 19) oriented perpendicular to the longitudinal direction of the wick axis and belonging to the heating arrangement designed as linear guide arrangement (20) is coupled to an electrical heating element (26).

8. Device according to Claim 7, **characterized in that**
the electrical heating element is a ceramic block (26) which is coupled to electrical lines (48) and which bears, via at least one ridge (27), on a support plate (25) connected to the at least one guide arm (18, 19) and can be held there by means of at least one locking arm (28) which is elastically mounted and prestressed in the direction of the ceramic block (26) and which engages laterally in an L-shape round the ceramic block (26), and
a wick recess (29) is formed in the ceramic block (26) and correspondingly in the support plate (25), through which recess (29) the wick end (30) can be received.

9. Device according to one of Claims 1 to 8, **characterized in that** a spacing and/or operation indicator visible from outside through a suitable housing aperture (39) is arranged on the linear guide arrangement (20).

10. Device according to one of Claims 1 to 9, **characterized in that**
the linear guide arrangement (20) is longitudinally displaceable between two end-stop positions (33, 34) formed by stops (31, 32) on the housing side, in such a way that
the linear guide arrangement (20), in a first end-stop position (33), is disposed directly in the area of the wick end (30) for a maximum degree of vaporization, and
the linear guide arrangement (20), in a second end-stop position (34), is disposed a distance above an upper wick end (30), as seen in the longitudinal direction of the wick axis, for a minimum degree of vaporization, said linear guide arrangement (20) also being able to be fixed in intermediate positions between the two end-stop positions (33, 34).

11. Device according to one of Claims 1 to 10, **characterized in that**
the housing (13) has ventilation slits (3) and is made up of a front housing shell (1) and a rear housing shell (7) which can be releasably connected to one another via correspondingly assigned catch elements (9, 10) of a catch connection (8), and
the rear housing shell (7) is formed by two housing half-shells (5, 6) which can be releasably connected to one another via correspondingly assigned catch elements (11) of a catch connection (12) in such a way that the linear guide arrangement (20) can be mounted on the housing (13), so as to be longitudinally displaceable in the longitudinal direction of the wick axis, after the housing half-shells (5, 6) have been assembled.

12. Device according to Claim 11, **characterized in that** the rear housing shell (7), in the assembled state, is designed with a double wall in an area below the linear guide arrangement (20) so as to form a cable channel (40).

13. Device according to Claim 12, **characterized in that**
a rear wall (41) of the rear housing shell (7) forming the outer wall of the housing in the double-walled housing area comprises a recess (42) for receiving a connector plug (43) for attachment to a socket in which the connector plug (43) can be fitted, in such a way that
the electrical lines (48) in the cable channel (40) are routed from the plug (43) to the heating arrangement designed as linear guide arrangement (20), the lines (48) being clamped in the cable channel (40) in such a way that the lines (48) are tensioned to some extent when the linear guide arrangement (20) is at the maximum distance from the wick end (30).

14. Device according to one of Claims 11 to 13, **characterized in that**, in a lower housing area, the housing (13) has a receiving slot (49) or receiving opening via which the container (50) can be inserted and/or screwed at least partially into the housing interior (51) and fixed releasably therein.

## Revendications

1. Dispositif de vaporisation de substances volatiles, en particulier d'insecticides et/ou de parfums,
avec un boîtier (13), comprenant un dispositif de chauffage (20), et
avec un récipient (30) susceptible d'être relié au boîtier (13), récipient prévu pour une substance à vaporiser, comprenant une mèche, associée à une extrémité de mèche (30), sortant du récipient (50), du dispositif de chauffage (20), le dispositif de chauffage (20) étant susceptible d'être déplacé par rapport à l'extrémité de mèche (30) et, ainsi, par rapport au boîtier (13), dans le but de régler le degré de vaporisation dans le cas d'un récipient (40) relié au boîtier (13), ceci de manière que le dispositif de chauffage (20), observé dans la direction longitudinale de l'axe de la mèche, puisse être fixé en différentes positions par rapport à l'extrémité de mèche (30),
**caractérisé en ce que**
le dispositif de chauffage est réalisé sous la forme de dispositif de guidage linéaire (20), monté sur le boîtier (13) de façon déplaçable longitudinalement par rapport à celui-ci, dans la direction longitudinale de l'axe de mèche.

2. Dispositif selon la revendication 1, **caractérisé en ce que** le dispositif de chauffage, réalisé sous la forme de dispositif de guidage linéaire (20), est susceptible d'être fixé sur le boîtier (13) au moyen d'une liaison à serrage et/ou d'une liaison à encliquetage (23, 24) susceptible d'être surchargée.

3. Dispositif selon la revendication 1 ou la revendication 2, **caractérisé en ce que** le dispositif de guidage linéaire (20) présente au moins un bras de guidage (18, 19), orienté perpendiculairement par rapport à l'axe de mèche, bras disposé avec une extrémité de bras de guidage (35, 36), pour un accès depuis le côté extérieur du boîtier dans un évidement en fente (14, 15) de la paroi de boîtier.

4. Dispositif selon la revendication 3, **caractérisé en ce que** le dispositif de guidage linéaire (20) présente deux bras de guidage (18, 19), montés sur des côtés de boîtier opposés

5. Dispositif selon la revendication 3 ou la revendication 4, **caractérisé en ce que** le dispositif de guidage linéaire (20) présente, sur chaque bras de guidage (18, 19), un premier élément de guidage linéaire (21, 22), orienté parallèlement à l'axe longitudinal de l'axe de mèche, et
**en ce que** le dispositif de guidage linéaire (20) présente sur le boîtier (13) un deuxième élément de guidage linéaire (16, 17), associé de manière correspondante à chaque premier élément de guidage linéaire (21, 22).

6. Dispositif selon la revendication 5, **caractérisé en ce que** le premier élément de guidage linéaire est une rainure de guidage linéaire (21, 22) et le deuxième élément de guidage linéaire est une nervure de guidage linéaire (16, 17), guidée à l'intérieur de celle-ci, et/ou
**en ce que**, sur la nervure de guidage linéaire (16, 17), sont réalisés des évidements d'encliquetage (23), espacés de manière correspondante à différentes positions d'espacement en direction longitudinale, évidements d'encliquetage dans lesquels s'engage une saillie d'encliquetage (24), réalisée dans la zone de la rainure de guidage linéaire (21, 22), saillie d'encliquetage prévue pour assurer une fixation, pouvant être surchargée, du dispositif de guidage linéaire (20) sous un espacement déterminé par rapport à l'extrémité de mèche (30).

7. Dispositif selon l'une des revendications 3 à 6, **caractérisé en ce que** le au moins bras de guidage (18, 19), orienté perpendiculairement par rapport à la direction longitudinale d'axe de mèche, du dispositif de chauffage, réalisé sous la forme de dispositif de guidage linéaire (20), est couplé à un élément chauffant (26) électrique.

8. Dispositif selon la revendication 7, **caractérisé en ce que**
l'élément chauffant électrique est un bloc en céramique (26), couplé à des lignes électriques (48) et reposant, par l'intermédiaire d'au moins une nervure (27), sur une plaque support (25) reliée au au moins un bras de guidage (18, 19) et y étant susceptible d'être fixé, à l'aide d'au moins un bras d'encliquetage (28), lié par la matière élastiquement et susceptible d'être précontraint dans la direction du bloc en céramique (26), le bras d'encliquetage (28) entourant latéralement en forme de L le bloc en céramique (26), et
**en ce que**, dans le bloc en céramique (26) et, de manière correspondante dans la plaque support (25), est réalisé un évidement pour mèche (29), à travers lequel l'extrémité de mèche (30) est susceptible d'être logée.

9. Dispositif selon l'une des revendications 1 à 8, **caractérisé en ce que**, sur le dispositif de guidage linéaire (20) est disposé un affichage d'espacement et/ou de fonctionnement, visible depuis l'extérieur, à travers un évidement de boîtier (39) correspondant.

10. Dispositif selon l'une des revendications 1 à 9, **caractérisé en ce que**
le dispositif de guidage linéaire (20) est déplaçable longitudinalement entre deux positions de butée de fin de course (33, 34) formées par deux butées (31, 32) situées côté boîtier,
de manière que le dispositif de guidage linéaire (20), en une première position de butée de fin de course (33), soit disposé pour un degré de vaporisation maximal dans la zone de l'extrémité de mèche (30), et
**en ce que**, en une deuxième position de butée de fin de course (34), le dispositif de guidage linéaire (20) est disposé pour un degré de vaporisation minimale, à distance au-dessus d'une extrémité de mèche supérieure (30), en observant dans la direction longitudinale de l'axe de mèche, le dispositif de guidage linéaire (20) étant susceptible d'être fixé également en des positions intermédiaires entre les deux positions de butée de fin de course (33, 34).

11. Dispositif selon l'une des revendications 1 à 10, **caractérisé en ce que** le boîtier (13) présente des fentes d'aération (3) et est construit en plusieurs parties, à partir d'une coque de boîtier avant (1) et d'une coque de boîtier arrière (7), reliées ensemble de façon désolidarisable par l'intermédiaire d'éléments d'encliquetage (9, 10), associés de manière correspondante, d'une liaison à encliquetage (8), et
**en ce que** la coque de boîtier arrière (7) est formée par de deux demi-coques de boîtier (5, 6), susceptibles d'être reliées ensemble de manière désolidarisable par l'intermédiaire d'éléments d'encliquetage (11), associés de manière correspondante, d'une liaison à encliquetage (12), de manière que le dispositif de guidage linéaire (20), après montage des demi-coques de boîtier (5, 6), soit monté sur le boîtier (13), de façon déplaçable longitudinalement, dans la direction longitudinale de l'axe de mèche.

12. Dispositif selon la revendication 11, **caractérisé en ce que** la coque de boîtier arrière (7), à l'état monté, est réalisée à double paroi dans une zone se trouvant au-dessous du dispositif de guidage linéaire (20), afin de former un canal à câbles ou goulotte (40).

13. Dispositif selon la revendication 12, **caractérisé en ce que**
une paroi arrière (14), formant, dans la zone de boîtier à paroi double la paroi extérieure de boîtier, paroi arrière appartenant à la coque de boîtier arrière (7), comprend un évidement (42) pour recevoir une prise de raccordement (43) pour le raccordement à une prise électrique, dans laquelle la prise de raccordement (43) est susceptible d'être insérée, de manière que
les lignes électriques (48), posées dans le canal à câble (40), soient guidées, en partant de la prise de raccordement (43) et en allant au dispositif de chauffage réalisé sous la forme de dispositif de guidage linéaire (20), sachant que les lignes (48), posées dans le canal à câble (40), sont serrées de manière que ces lignes (48) soient quelque peu serrées par l'extrémité de mèche (30) lorsqu'on est à distance maximale du dispositif de guidage linéaire (20).

14. Dispositif selon l'une des revendications 11 à 13, **caractérisé en ce que** le boîtier (13) présente, dans une zone de boîtier inférieur, une fente de logement (49) ou une ouverture de logement, à travers laquelle le récipient (50) peut être introduit, au moins partiellement, dans l'espace intérieur de boîtier (51 ) et/ou y être vissé et/ou y être fixé de façon désolidarisable.
